# EUROPÄISCHE PATENTSCHRIFT

(11) **EP 1 545 290 B1**
(45) Veröffentlichungstag und Bekanntmachung des Hinweises auf die Patenterteilung: **31.10.2007**
(21) Anmeldenummer: 03769316.5
(22) Anmeldetag: 25.09.2003
(51) Int. Cl.: A61B 1/07, G02B 23/24

(54) **ENDOSKOP MIT KOMBINIERTEM BELEUCHTUNGS- UND BILDÜBERTRAGUNGSSYSTEM**
ENDOSCOPE PROVIDED WITH A LIGHTING SYSTEM AND A COMBINE IMAGE TRANSMISSION
ENDOSCOPE COMPORTANT UN SYSTEME D'ECLAIRAGE ET DE TRANSMISSION D'IMAGES COMBINE

(30) Priorität: 05.10.2002 DE 10246521
(43) Veröffentlichungstag der Anmeldung: 29.06.2005
(73) Patentinhaber: Karl Storz GmbH & Co. KG, 78532 Tuttlingen (DE)
(72) Erfinder: IRION, Klaus, 78576 Liptingen (DE)
(74) Vertreter: Stamer, Harald
(86) Internationale Anmeldenummer: PCT/EP2003/010653
(87) Internationale Veröffentlichungsnummer: WO 2004/032731

(56) Entgegenhaltungen:
- WO-A-91/15793
- DE-A- 19 639 653
- US-A- 5 298 741
- US-A- 2002 087 047
- PATENT ABSTRACTS OF JAPAN vol. 2002, no. 05, 3. Mai 2002 (2002-05-03) & JP 2002 023067 A (FUJI PHOTO OPTICAL CO LTD), 23. Januar 2002 (2002-01-23) in der Anmeldung erwähnt
- PATENT ABSTRACTS OF JAPAN vol. 1999, no. 04, 30. April 1999 (1999-04-30) & JP 11 019026 A (FUJI PHOTO FILM CO LTD), 26. Januar 1999 (1999-01-26)

## Beschreibung

Die Erfindung betrifft ein Endoskop zur Beleuchtung und Beobachtung von Objektfeldern in Hohlräumen mit einer Beleuchtungseinheit und einem Bildübertragungssystem, dem distalseitig ein Objektiv und proximalseitig eine Okular- oder Kameraoptik als Beobachtungssystem zugeordnet sind.

Endoskope dieser Art in starrer und flexibler Ausgestaltung sind bekannt und z.B. in dem Aufsatz "Optical Principles of Endoscopy", R. Prescott, J. Med. Primatol.5:133-147 (1976) mit ihren grundsätzlichen Eigenschaften beschreiben. Alle bisher bekannten Endoskope dieser Art weisen neben dem Übertragungssystem zur Bilderfassung und Bildübertragung mindestens ein davon getrenntes System zur Lichtübertragung für die Beleuchtung des zu beobachtenden Objektfeldes auf. Das Objektfeld kann sich im Hohlraum eines technischen oder eines biologischen Objekts befinden.

Vor allem bei kleinkalibrigen Endoskopen, den sogen. Miniendoskopen, führen diese getrennten Übertragungssysteme zu einer überproportionalen Erhöhung des Durchmessers. Das hängt vor allem auch damit zusammen, daß die beiden Übertragungssysteme gegeneinander sowohl optisch als auch mechanisch isoliert werden müssen, um einerseits ein Übersprechen des Lichts vom Beleuchtungssystem zum Bildübertragungssystem zu verhindern und andererseits ein stabiles und autoklavfestes Gesamtsystem zu erhalten.

Die dünnsten Bildübertragungssysteme liegen bereits im Bereich von 200 µm und besitzen eine Auflösung von ca. 6000 Bildelementen.
Erstellt man auf dieser Basis ein konventionelles Miniendoskop, so wird ein zusätzliches Lichtleitsystem notwendig, das optisch isoliert, z.T. koaxial, zum Bildleiter adaptiert wird. Meist wird das koaxial aufgebaute System noch durch eine Ummantelung zur mechanischen Stabilität, Fixierung der Lichtleitfasern und der Autoklavierbarkeit umschlossen. Der Durchmesser des gesamten Miniendoskops kommt mit diesen Maßnahmen auf mehr als das doppelte des Durchmessers des Bildleiters. Bei größeren Endoskopen ist dieses Verhältnis nicht ganz so extrem, aber auch hier kommt es zu einer signifikanten Erhöhung durch den getrennten Lichtkanal. Bei einem starren Endoskop mit Stablinsen besitzt das Linsensystem z.B. einen Durchmesser von ca. 2,8 mm und das Gesamtsystem mit Licht und mechanischen Ummantelungen ca. 4,0 mm.

Für viele Applikationen besteht der Wunsch, das Verhältnis zwischen Bildleiter und Gesamtdurchmesser des endoskopischen Systems zu verbessern. Insbesondere in der Zahnmedizin bestehen Anwendungen, bei denen extrem dünne und flexible Endoskope notwendig sind, z.B. bei der Inspektion des künstlich eröffneten Zahnwurzelkanals bis zum Apex oder bei der Inspektion des Spinalkanals.

Aus DE 196 39 653 A1 sind Endoskope zur Beobachtung von biologischem Gewebe bekannt, die zur Fluoreszenzendoskopie eingesetzt werden. Es wird Licht mit einer kurzwelligen Anregung appliziert und Fluoreszenzlicht einer langwelligeren Emission als Bild erfaßt und endoskopisch weitergeleitet. Das Fluoreszenzlicht enthält Informationen, die zu einer Gewebecharakterisierung verwendet werden können. Das Anregungslicht wird über einen separaten Lichtleiter parallel zur Übertragung des Fluoreszenzlichts auf das Gewebe eingestrahlt. Die Transmissionsgrade im lichtzuführenden und im bilderzeugenden Teil sind so gewählt, daß das Bild des mit Anregungslicht beleuchteten Gewebebereichs gleichzeitig mittels Fluoreszenzlicht und reflektiertem Beleuchtungslicht erzeugt wird, wobei die beiden zur Bilderzeugung beitragenden Anteile bezüglich ihrer Wellenlänge und bezüglich ihrer Intensität so beschaffen sind, daß keine gegenseitige Störung auftritt.

Auch bei dieser Anwendung besteht ein generelles Entwicklungsziel darin, die Endoskopdurchmesser weiter zu reduzieren, um ein hinsichtlich einer geringeren Traumatisierung des Patienten dünneres Instrumentarium zu besitzen. Dem steht dann aber häufig entgegen, daß die über die Lichtleiter eingestrahlte Lichtintensität für eine ausreichende Fluoreszenzanregung nicht mehr groß genug ist und die über den Bildleiter erfaßte Fluoreszenzstrahlung für die Empfindlichkeit des Nachweissystems nicht ausreicht.

Andere Systeme zur Untersuchung von Körperhöhlen weisen ein aus Objektiv und Okular bestehendes Abbildungssystem auf, mit dem nur relativ kurze Abbildungswege überbrückt werden können.

Aus DE 1 746 649 U ist ein Kolpomikroskop zur Beobachtung von Vorgängen in Körperhöhlen lebender Organismen bekannt. Das Gerät enthält eine Beleuchtungseinheit, ein Objektiv und ein Okular. Das Beleuchtungslicht wird über ein Interferenz-Spiegelfilter in den Beobachtungsstrahlengang zwischen Objektiv und Okular eingekoppelt. Zur Fluoreszenzbeobachtung wird in die Beleuchtungseinheit ein Blaufilter eingefügt und dem Okular ein Sperrfilter für Reste der blauen Erregerstrahlung vorgeschaltet. Zur Auflichtbeobachtung wird in die Beleuchtungseinheit ein Polarisationsfilter eingefügt und dem Okular ein Polarisations-Sperrfilter für die innerhalb des Gerätes reflektierten Anteile der polarisierten Beleuchtungsstrahlen vorgeschaltet. Das in die Körperhöhle einzuführende Rohr mit dem Objektiv ist sehr kurz und hat einen relativ großen Durchmesser.

Aus DE 35 42 207 A1 ist eine Einrichtung zur Durchführung medizinischer Untersuchungen in Körperhöhlen bekannt. Die Einrichtung enthält eine auf das zu untersuchende Aufnahmefeld ausgerichtete Video-Kamera. In einer Ausführungsform wird das Licht zur Beleuchtung des Aufnahmefeldes über einen der Kamera vorgeschalteten halbdurchlässigen Spiegel koaxial zur optischen Achse auf das Objekt gelenkt. Dem halbdurchlässigen Spiegel kann ein Polarisationsfilter zur Erzielung spezieller Effekte nachgeordnet sein. Das Beleuchtungsystem ist in einem kurzen Rohr untergebracht, das auf die Kamera aufgesetzt ist und einen relativ großen Durchmesser aufweist.

Aus JP 2002-023 067 A ist ein optisches System für ein elektronisches Endoskop bekannt. Das System enthält objektseitig ein Objektivsystem, dem eine λ/4-Platte und ein Polarisator vorgeschaltet sind. Der Polarisator ist aus zwei rechtwinkligen Prismen zu einem Würfel zusammengesetzt, wobei die aufeinander liegenden Flächen der Prismen eine Polarisationsfolie einschließen. Dem Polarisator wird über einen Lichtleiter das Beleuchtungslicht zugeführt, das an der Polarisationsfolie linear und durch die λ/4-Platte zirkular polarisiert wird. Die vom Objekt reflektierte und vom Objektiv aufgenommene Strahlung wird in bekannter Weise durch die λ/4-Platte senkrecht zur beleuchtenden Strahlung wieder linear polarisiert und an der Polarisationsfolie in Richtung auf eine elektronische Kamera reflektiert. Der Durchmesser des in die Körperhöhle einzuführenden Systemteiles ist aufgrund der Dimensionierung des Polarisators und der daran angrenzenden Kamera relativ groß.

Aus US 2002/0087047 A1 ist ein Miniatur-Endoskopsystem mit einem optischen System bekannt, das sowohl zur Übertragung des Beleuchtungslichts als auch zur Bildübertragung dient. Dem optischen System ist proximal ein Strahlenteiler mit komplementär linear polarisierenden Reflexions- und Transmissionseigenschaften vorgeschaltet. Das optische System besteht aus einem relativ kurzen dünnen Rohr mit einer einzigen distalseitig eingesetzten Linse. Die Bildübertragung erfolgt ohne Zwischenbild im Rohr. Die Krümmung der Linsenflächen ist so gewählt, daß die Intensität des rückgestreuten Streulichts möglichst gering ist. Zusätzlich ist die Innenwand des Rohres zur Absorption von Streulicht beschichtet. Zur weiteren Streulichtunterdrückung bei der Abbildung des beobachteten Objekts sind im Beleuchtungs- und Abbildungsstrahlengang zueinander gekreuzt ausgerichtete Polarisatoren angeordnet.

Der Erfindung lag daher die Aufgabe zugrunde, ein im Durchmesser weiter reduziertes Endoskop zu schaffen, bei dem eine von außen zugeführte Beleuchtung mit ausreichender Intensität möglich ist, ohne den für die Bildübertragung notwendigen Durchmesser des Bildübertragungssystems einschränken zu müssen. Dabei sollte die Trennschärfe zwischen Beleuchtungs- und Abbildungsstrahlen auch bei komplexen Bildübertragungssystemen mit längeren Abbildungswegen verbessert werden.

Diese Aufgabe wird bei einem Endoskop der eingangs genannten Art erfindungsgemäß dadurch gelöst, daß proximalseitig ein optisches Teilerelement für zueinander komplementäre Licht-Polarisationen oder für zueinander komplementäre Wellenlängenbereiche und Licht-Polarisation zwischen die Beleuchtungseinheit, das Bildübertragungssystem und das Beobachtungssystem derart eingefügt ist, daß das von der Beleuchtungseinheit erzeugte Beleuchtungslicht in das Bildübertragungssystem einkoppelbar ist. Dabei wird es sich im allgemeinen nicht vermeiden lassen, daß aufgrund einer ungenügenden Trennschärfe des Teilerelements auch Anteile des Beleuchtungslichts in das Beobachtungssystem eintreten. Analog zu der aus DE 196 39 653 A1 bekannten Fluoreszenzendoskopie ist das sogar ein Vorteil. Grundsätzlich wird jedoch eine möglichst vollständige Trennung der zueinander komplementären Lichtanteile angestrebt.

Durch die distalseitige Einfügung eines λ/4-Plättchens vor dem Objektiv wird die Wirkung der Trennung nach Licht-Polarisationen am Teilerelement wesentlich verbessert.

Wenn das Teilerelement für komplementäre Licht-Polarisationen optimiert ist, dann können zusätzliche Spektralfilter für den kurzwelligen und den längerwelligen Spektralbereich vorgesehen sein. Selbstverständlich können spezielle Spektralfilter auch zur Erhöhung der Trennschärfe eines spektral und polarisationsoptisch trennenden Teilerelements und spezielle Polarisationsfilter zur Erhöhung der Trennschärfe eines rein polarisationsoptischen Teilerelements oder eines spektral und polarisationsoptischen Teilerelements vorgesehen sein.

Andere vorteilhafte Ausgestaltungen der Erfindung ergeben sich aus den Merkmalen der weiteren Unteransprüche.

Durch die Anordnung des Teilerelements steht für die Übertragung des Beleuchtungslichts nunmehr der gesamte Querschnitt des Bildübertragungssystems zur Verfügung. Da dieser üblicherweise größer als der Querschnitt eines sonst üblichen separaten Lichtübertragungssystems ist, werden die Bedingungen für die Übertragung einer ausreichenden Lichtintensität zur Fluoreszenzanregung wesentlich verbessert. Durch den Wegfall eines separaten Lichtleitsystems für die Beleuchtung kann der Querschnitt des Bildübertragungssystems für die nachzuweisende Bildinformation optimiert werden. Durch die Auftrennung in komplementäre Licht-Polarisationen am Teilerelement wird eine wirksame Trennung der Beleuchtungseinheit vom Beobachtungssystem gewährleistet.

Ein endoskopisches Faserbündelsystem mit einem vorgeschalteten dichroitischen Strahlenteiler ist aus US 5 298 741 A grundsätzlich bekannt. Es wird eine Anordnung beschrieben, bei der auf dem distalen Ende des Faserbündelsystems ein oder mehrere Fluoreszenzstoffe oberflächlich aufgetragen sind, die mit der angrenzenden Körperflüssigkeit bzw. einem Gewebebestandteil eine Reaktion eingehen und somit zu einer konzentrationsabhängigen Fluoreszenz angeregt werden, wenn sie mit Licht einer geeigneten Wellenlänge bestrahlt werden. Das Anregungslicht wird durch Anregungsfilter ausgewählt und über einen dichroitischen Teiler in das Faserbündelsystem eingekoppelt. Das am distalen Ende des Faserbündelsystems entstehende Fluoreszenzlicht wird über dasselbe Faserbündelsystem zurück zum proximalen Ende übertragen und über den dichroitischen Teiler und ein weiteres geeignetes Nachweisfilter einem Auswertungssystem zugeleitet. Zur endoskopischen Beobachtung eines Hohlraumes ist das System nicht geeignet.

Überraschenderweise hat sich gezeigt, daß bei proximalseitiger Anordnung eines optischen Teilerelements für komplementäre Licht-Polarisationen sowohl das notwendige Licht zur Beleuchtung in der einen Richtung als auch das aus dem Hohlraum kommende Licht zur Beobachtung in der anderen Richtung übertragen und nach der Trennung am Teilerelement ohne gegenseitige Störung ausgewertet und beobachtet werden kann. Als Bildübertragungssysteme können dabei mit Vorteil Multifaser-Bildleitersysteme mit planen Endflächen, Bildübertragungssysteme aus Stablinsen mit gekrümmten Endflächen oder auch Gradientenindex-Stablinsen mit planen Endflächen vorgesehen sein.

Die Verwendung komplementärer Wellenlängenbereiche für Beleuchtung und Beobachtung ist besonders vorteilhaft bei Verfahren der Fluoreszenzdiagnose am biologischen Gewebe. Die Beleuchtung erfolgt dabei üblicherweise mit Wellenlängen vom nahen UV bis 430 oder 450 nm und die Beobachtung in dem jeweils daran anschließenden längerwelligen Spektralbereich.

Andererseits kann es auch wünschenswert sein, das Objektfeld zusätzlich im Weißlicht beobachten zu können. Das für eine Aufhellung des Objektfeldes notwendige zusätzliche Beleuchtungslicht kann dazu in üblicher Weise über ein separates, relativ dünn-kalibriges faseroptisches Beleuchtungssystem an das distale Ende des Endoskops geführt werden. Zur Beobachtung im Weißlicht kann dann proximalseitig zwischen dem Bildübertragungssystem und dem Teilerelement ein Neutralteiler mit geeignetem Teilerverhältnis eingefügt sein, der einen Teil des zurückkommenden Lichts aus dem Strahlengang zur Beobachtung auskoppelt.

Sollen Beleuchtung und Beobachtung jedoch im selben Wellenlängenbereich erfolgen, so können das Beleuchtungs- und das Beobachtungslicht zueinander senkrecht linear polarisiert sein. Zur Vermeidung störender Reflexe ist es dabei vorteilhaft, dem distalen Ende des Bildübertragungssystems ein λ/4-Plättchen vorzuschalten. Dieses erzeugt in an sich bekannter Weise aus linear polarisiertem Licht zirkular polarisiertes Licht. Nach Reflexion an dem zu beobachtenden Gewebe wird das zirkular polarisierte Licht am λ/4-Plättchen in 90° gedrehtes linear polarisiertes Licht umgewandelt, das am Teilerelement zur Beobachtung ausgekoppelt wird.

Üblicherweise ist das Beleuchtungslicht im Verhältnis zum Beobachtungslicht wesentlich heller. Reflexionen des Beleuchtungslichts an Glas-/Luftflächen oder Kittflächen innerhalb des Bildübertragungssystems können daher in der derselben Größenordnung liegen wie das am Gewebe reflektierte Beobachtungslicht. Für eine optimale Trennung des Beobachtungslichts vom Beleuchtungslicht ist es daher vorteilhaft, das Teilerelement sowohl für eine Trennung nach Wellenlängenbereichen als auch nach Licht-Polarisation auszulegen.

Das optische Teilerelement kann in an sich bekannter Weise mit Vorteil aus zwei gleichseitigen Rechtwinkelprismen zu einem Würfel zusammengesetzt sein, bei dem die Hypotenusenfläche eines der Rechtwinkelprismen mit einer polarisationsoptischen oder einer spektralen und polarisationsoptischen Filterschicht belegt ist und wobei je eine geeignete Kathetenfläche der Beleuchtungseinheit, dem Bildübertragungssystem und dem Beobachtungssystem zugeordnet ist.

Den Kathetenflächen können dabei ergänzende Polarisations- oder Spektral-Filterelemente zugeordnet sein.

Bei Verwendung eines Bildübertragungssystems mit einer proximal planen Endfläche kann der Würfel mit der zugehörigen Kathetenfläche auf diese Endfläche aufgesetzt werden. Der Kathetenfläche für das Beobachtungssystem kann ein Flächen-Bildsensor mit integriertem halbleiterbasierten Ladungs- bzw. Elektronenvervielfacher und evtl. einem Kühlelement zugeordnet sein. Der Kathetenfläche für die Beleuchtungseinheit kann eine Laserdiodenmatrix als Lichtquelle zugeordnet sein. Eine solche Anordnung der Beleuchtungs- und Beobachtungsmittel am Teilerelement erlaubt einen äußerst kompakten Aufbau des Endoskops.

Insbesondere können auch die Beleuchtungseinheit, das Teilerelement und das Beobachtungssystem in einer Gehäuseeinheit zusammengefaßt sein, die mit einem Adapter zum auswechselbaren Ansetzen des Bildübertragungssystems an die zugehörige Kathetenfläche des Teilerelements versehen ist. Auf diese Weise können auch Einweg-Bildübertragungssysteme verwendet werden. Als Lichtquellen können vorzugsweise Xenon-, Quecksilberdampf- oder Halid-Lampen oder auch ein Lasersystem in die Gehäuseeinheit eingesetzt werden. Insbesondere mit Hilfe eines Lasersystems können die für die photodynamische Therapie (PDT), d.h. für die unter visueller Beobachtung gezielte Zerstörung von Gewebe, notwendigen hohen Lichtstärken erzeugt werden.

Zur Anpassung an unterschiedliche Beobachtungsverfahren kann auch das Teilerelement auswechselbar in der Gehäuseeinheit angeordnet sein.

Ausführungsbeispiele sind in der Zeichnung schematisch dargestellt und werden nachfolgend anhand der Figuren beschrieben. Dabei zeigen:
- Fig.1a: ein Endoskop mit Teilerelement,
- Fig.1b: ein Endoskop mit Teilerelement und zusätzlichen Filterelementen,
- Fig.1c: ein Endoskop mit zusätzlicher Weißlicht-Aufhellung,
- Fig.2: ein Ausführungsbeispiel mit Gehäuseeinheit,
- Fig.3: eine andere Ausführungsform des Endoskops und
- Fig.4: eine Ausführungsform mit zusätzlicher Weißlicht-Aufhellung.

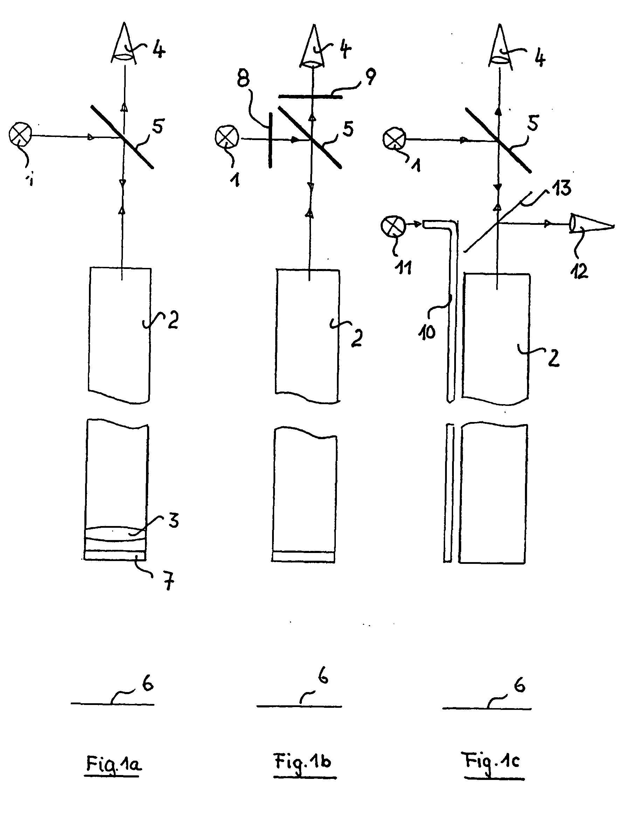

In Fig.1 ist das Grundprinzip eines Endoskops mit einem erfindungsgemäß eingefügten Teilerelement in unterschiedlichen Varianten dargestellt.

Das in Fig.1a gezeigte Endoskop besteht zunächst aus einer Beleuchtungseinheit 1 und einem Bildübertragungssystem 2, dem distalseitig ein Objektiv 3 und proximalseitig ein Beobachtungssystem 4 zugeordnet sind. Proximalseitig ist weiterhin ein Teilerelement 5 derart angeordnet, daß einerseits das von der Beleuchtungseinheit 1 erzeugte Beleuchtungslicht in das Bildübertragungssystem 2 einkoppelbar ist und andererseits das an einem Objektfeld 6 in einem Hohlraum reflektierte und über das Bildübertragungssystem 2 zurückgeleitete Licht dem Beobachtungssystem 4 zugeführt wird. Dabei hat das Teilerelement 5 die Eigenschaft, zueinander komplementäre Licht-Polarisationen oder Wellenlängenbereiche und Licht-Polarisationen in Reflexion und Transmission unterschiedlich zu beeinflussen. Soweit das Teilerelement 5 in Reflexion und Transmission jeweils für eine Trennung senkrecht zueinander ausgerichteter Linearpolarisationen ausgelegt ist, ist es vorteilhaft, dem Objektiv 3 distalseitig ein λ/4-Plättchen 7 vorzuschalten.

Bei dem Endoskop nach Fig.1b sind dem Teilerelement 5 beleuchtungsseitig und beobachtungsseitig ergänzende Filterelemente 8, 9 zugeordnet. Dabei kann das Teilerelement 5 z.B. für die Trennung komplementärer Licht-Polarisationen und Wellenlängenbereiche optimiert sein. In diesem Fall können das beleuchtungsseitige Filterelement 8 als Polarisator und das beobachtungsseitige Filterelement 9 als Analysator ausgebildet sein. Ist dagegen das Teilerelement 5 für die Trennung komplementärer Licht-Polarisationen optimiert, so kann das Filterelement 8 vorzugsweise als Spektralfilter für einen kurzwelligen Spektralbereich und das Filterelement 9 für einen längerwelligen Spektralbereich ausgebildet sein, wenn die Anordnung für eine Fluoreszenzanalyse eingesetzt werden soll.

Bei der Anordnung nach Fig.1c ist ein zusätzliches faseroptisches Beleuchtungssystem 10 mit einer Weißlichtquelle 11 zur Aufhellung des in dem Hohlraum zu beobachtenden Objektfeldes 6 vorgesehen. Dem vom Objektfeld 6 reflektierten und vom Bildübertragungssystem 2 zurückgeleiteten Licht ist daher ein Weißlichtanteil überlagert, der z.B. zusätzlich die Umgebung eines zur Fluoreszenz angeregten Objektdetails sichtbar macht. Um dieses Bild einer Beobachtung 12 zugänglich zu machen, ist ein Neutralteiler 13 am proximalseitigen Ausgang des Bildübertragungssystems 2 vorgesehen. Am nachfolgenden Teilerelement 5 wird der Weißlichtanteil bis auf die evtl. vorhandenen längerwelligen Spektralanteile unterdrückt, die in den Spektralbereich der Fluoreszenzanregung fallen.

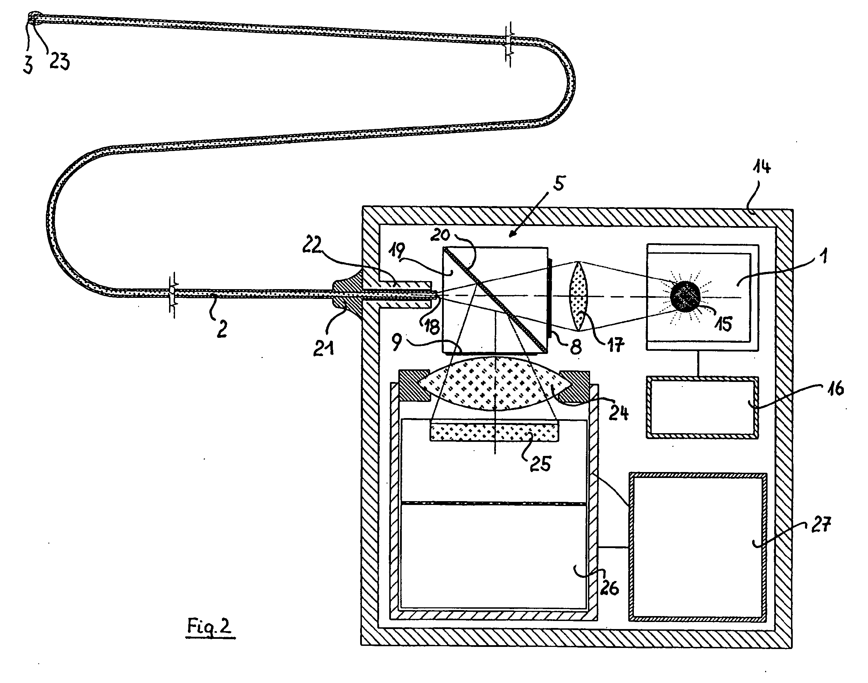

Bei dem in Fig.2 dargestellten Ausführungsbeispiel sind in einer Geräteeinheit 14 eine Beleuchtungseinheit 1 mit Lampe 15 und Lampensteuereinheit 16, sowie einer Kollektorlinse 17 angeordnet. Als Lampe 15 können insbesondere eine Xenon-, Quecksilberdampf- oder Halid-Lampe oder auch ein Laser- oder Laserdiodensystem vorgesehen sein. Die Kollektorlinse 17 bildet die Strahlung der Lampe 15 durch ein Teilerelement 5 hindurch auf eine proximale Endfläche 18 eines Bildübertragungssystems 2 ab.

Das Teilerelement 5 besteht aus zwei gleichseitigen Rechtwinkelprismen 19, die mit ihren Hypotenusenflächen zusammengesetzt sind und somit einen Würfel bilden. Die Hypotenusenflächen der Rechtwinkelprismen 19 sind mit einer polarisationsoptischen oder spektralen und polarisationsoptischen Teilerschicht 20 belegt. Die spektrale Teilerschicht 20 soll z.B. für einen vorbestimmten Wellenlängenbereich aus dem Emissionspektrum der Lampe 15 durchlässig und für einen dazu komplementären, i.a. längerwelligen, Wellenlängenbereich reflektierend sein. Der reflektierte Strahlungsanteil wird gegen die Wandung der Gehäuseeinheit 14 gelenkt und dort z.B. in einer matt schwarzen Beschichtung als Strahlenfalle absorbiert.

Als polarisationsoptische Teilerschicht kann z.B eine Polarisationsfolie vorgesehen sein, die die durch die Folie hindurchtretende Strahlung der Lampe 15 linear polarisiert. Eine gute Polarisation kann über einen weiten Wellenlängenbereich erzeugt werden, so daß in das Bildübertragungssystem 2 quasi Weißlicht eingekoppelt wird. Selbstverständlich ist auch eine Kombination in der spektralen Begrenzung des Wellenlängenbereichs und der linearen Polarisation möglich, die der spektralen Abhängigkeit der Polarisationsfolien grundsätzlich sogar entgegen kommt. Die Teilerschicht 20 kann auch mit je nach Anwendungszweck ausgewählten ergänzenden Filterelementen 8, 9 kombiniert werden.

Das Bildübertragungssystem 2 kann vorzugsweise als Multifaser-Bildleiter ausgebildet sein. Ein solches System ist relativ flexibel und besitzt im allgemeinen eine hohe spektrale Transmission, wenn die Fasern aus Quarzglas hergestellt sind. Daher können auch größere Längen ohne signifikante Dämpfung bei der Lichtübertragung hergestellt werden. Die Systeme haben im Vergleich zum Gesamtdurchmesser eine relativ gute Bildqualität. Das Quarzglas weist darüber hinaus keine Eigenfluoreszenz auf. Die Endflächen sind im allgemeinen plan.

Die realisierbare Länge des Multifaser-Bildleiters kann so gewählt werden, daß die proximale Endfläche außerhalb des sterilen Patientenbereichs liegt. Aufgrund der relativ geringen Kosten des Multifaser-Bildleiters durch seine einfache und automatisierbare Herstellung ist es daher vorteilhaft, den Bildleiter von der Geräteeinheit 14 entkoppelbar anzuordnen und als Einwegprodukt zu verwenden. Damit lassen sich die aus jedem Einsatz entstehenden Reinigungs-, Sterilisations- und Kontaminationsprobleme vermeiden.

Im Ausführungsbeispiel der Fig.2 ist das Bildübertragungssystem 2 am proximalen Ende mit einem Steckanschlag 21 versehen, durch den es in einem Adapter 22 in der Gehäuseeinheit 14 in seiner Lage gegenüber einer Kathetenfläche des Teilerelements 5 fixiert wird.

Das Bildübertragungssystem 2 ist an seiner distalen Endfläche 23 mit einem Objektiv 3 versehen. Das Objektiv 3 kann z.B. als Gradienten-Linse ausgeführt sein, die auf die Endfläche 23 aufgeschweißt ist, um Eigenfluoreszenz durch einen Kleber zu unterbinden. Bei Verwendung von linear polarisiertem Licht kann auf die plane Endfläche 23 ein nicht dargestelltes λ/4-Plättchen aufgesetzt werden.

Das über das Objektiv 3 ausgekoppelte Beleuchtungslicht leuchtet einen bestimmten Winkelbereich vor dem Objektiv 3 aus. Das System ist so ausgelegt, daß über das distalseitige Objektiv 3 ein Bild mit einem Bildwinkel erfaßt wird, der zumindest nahezu dem Ausleuchtwinkel entspricht.

Das vom Objektiv 3 aufgenommene Bild besteht im Falle der Fluoreszenzdiagnose aus einer Strahlung mit einer Wellenlänge, die komplementär zum Beleuchtungslicht ist. Die dem Bild zugehörige Strahlung wird an der Teilerschicht 20 reflektiert und dem Beobachtungssystem zugeführt. Ebenso wird bei linearer Polarisation des Beleuchtungslichts die dem Bild zugehörige Strahlung dazu senkrecht polarisiert sein und daher ebenfalls an der Teilerschicht 20 reflektiert.

Das dargestellte Beobachtungssystem besteht aus einer Feldlinse 24 und z.B. einem Flächen-Bildsensor 25 nach dem Prinzip der Single-Photon-Detektion (SPD-Prinzip). Dieser Bildsensor hat den Vorteil, daß die Mittel zur Bildverstärkung in den Halbleiter-Chip integriert sind, so daß ein leichter und kompakter Aufbau für die Bildaufnahme möglich ist. Im Zusammenwirken mit der Bildübertragung durch einen Multifaser-Bildleiter weist ein zweidimensionaler SPD-Bildaufnehmer noch den Vorteil auf, daß die durch jede einzelne Bildfaser entstehenden Bildpunkte sich mit daneben liegenden Bildpunkten teilweise überlagern, wodurch ein gleichmäßigeres Bild entsteht.

Dem SPD-Bildsensor 25 ist noch eine Kühleinrichtung 26 zugeordnet, die z.B. ein Peltierelement oder auch ein Behälter mit gekühltem CO₂ sein kann. Zur Ansteuerung des Bildsensors 25 und der Kühleinrichtung 26 ist in der Gehäuseeinheit 14 noch eine Steuereinrichtung 27 vorgesehen. Diese kann auch einen nicht dargestellten Monitor zur visuellen Bildbetrachtung ansteuern.

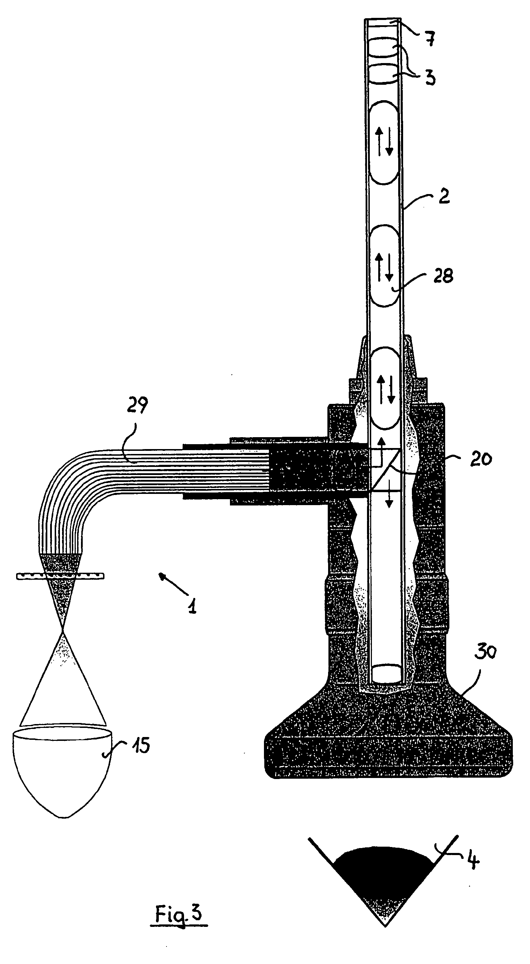

Bei dem in Fig.3 dargestellten Ausführungsbeispiel besteht das Bildübertragungssystem 2 aus Stablinsen 28 mit im allgemeinen gekrümmten Endflächen. Ihr Vorteil ist die sehr gute Bildqualität mit einer guten Schärfentiefe und hoher Lichttransmission. Die Stablinsen sind jedoch in der Herstellung aufwendig, nicht beliebig dünn ausführbar und besitzen häufig Kittflächen, die zur Eigenfluoreszenz neigen.

Diese Nachteile können vermieden werden, wenn das Bildübertragungssystem mit Stablinsen aus einem Gradientenindex-Material mit planen Endflächen aufgebaut wird.

Die Beleuchtungseinheit 1 enthält ein Lichtleiterbündel 29, das seitlich an ein Einblickgehäuse 30 angesetzt ist, so daß das Beleuchtungslicht an der Teilerschicht 20 reflektiert und die Beobachtung 4 in Transmission erfolgt.

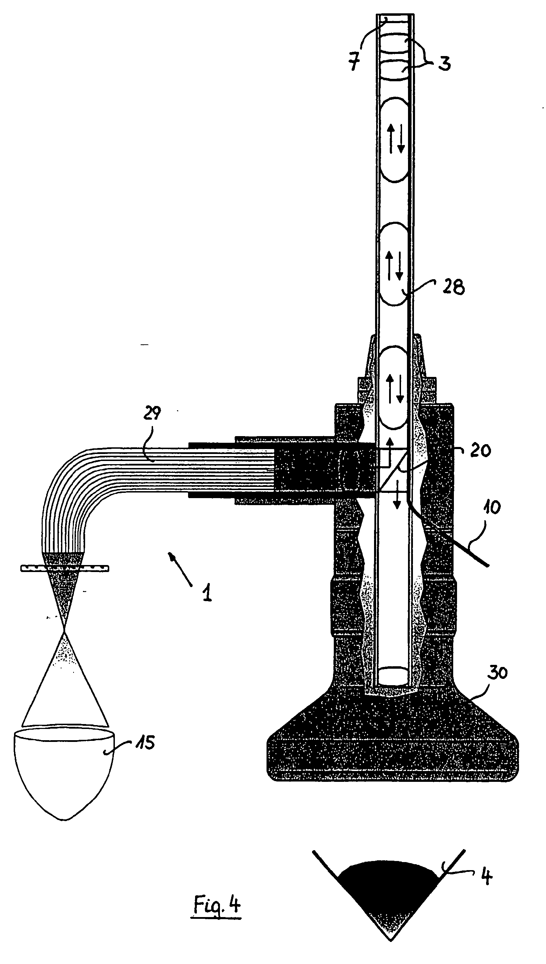

Das in Fig.4 dargestellte Ausführungsbeispiel entspricht dem vorhergehenden. Hier ist jedoch zusätzlich ein faseroptisches Beleuchtungssystem 10 für Weißlichtbeleuchtung eingefügt. Die Dimensionierung des Faserbündels 10 im Vergleich zum Bildübertragungssystem der Stablinsen 28 macht deutlich, daß der Gesamtquerschnitt des in eine Körperhöhle einzuführenden Endoskopteiles hier nicht wesentlich beeinflußt wird.

### Bezugszeichenliste

- 1: Beleuchtungseinheit
- 2: Bildübertragungssystem
- 3: Objektiv
- 4: Beobachtungssystem
- 5: Teilerelement
- 6: Objektfeld
- 7: λ/4-Plättchen
- 8: beleuchtungsseitiges Filterelement
- 9: beobachtungsseitiges Filterelement
- 10: faseroptisches Beleuchtungssystem
- 11: Weißlichtquelle
- 12: Weißlichtbeobachtung
- 13: Neutralteiler
- 14: Gehäuseeinheit
- 15: Lampe
- 16: Lampensteuereinheit
- 17: Kollektorlinse
- 18: proximale Endfläche
- 19: Rechtwinkelprisma
- 20: Teilerschicht
- 21: Steckanschlag
- 22: Adapter
- 23: distale Endfläche
- 24: Feldlinse
- 25: Flächenbildsensor
- 26: Kühleinrichtung
- 27: Steuereinrichtung
- 28: Stablinse
- 29: Lichtleiterbündel
- 30: Einblickgehäuse

## Patentansprüche

1. Endoskop zur Beleuchtung und Beobachtung von Objektfeldern (6) in Hohlräumen mit einer Beleuchtungseinheit (1) und einem endoskopischen Bildübertragungssystem (2), dem distalseitig ein Objektiv (3) und proximalseitig eine Okular- oder Kameraoptik als Beobachtungssystem (4) zugeordnet sind, das proximalseitig ein optisches Teilerelement (5) enthält, das zwischen die Beleuchtungseinheit (1), das Bildübertragungssystem (2) und das Beobachtungssystem (4) derart eingefügt ist, daß das von der Beleuchtungseinheit (1) erzeugte Beleuchtungslicht in das Bildübertragungssystem (2) einkoppelbar ist, wobei das optische Teilerelement (5) bezüglich Reflexion und Transmission für zueinander komplementäre Licht-Polarisationen ausgelegt ist, **dadurch gekennzeichnet, daß** distalseitig dem Objektiv (3) ein λ/4-Plättchen (7) vorgeschaltet ist.

2. Endoskop zur Beleuchtung und Beobachtung von Objektfeldern (6) in Hohlräumen mit einer Beleuchtungseinheit (1) und einem endoskopischen Bildübertragungssystem (2), dem distalseitig ein Objektiv (3) und proximalseitig eine Okular- oder Kameraoptik als Beobachtungssystem (4) zugeordnet sind, das proximalseitig ein optisches Teilerelement (5) enthält, das zwischen die Beleuchtungseinheit (1), das Bildübertragungssystem (2) und das Beobachtungssystem (4) derart eingefügt ist, daß das von der Beleuchtungseinheit (1) erzeugte Beleuchtungslicht in das Bildübertragungssystem (2) einkoppelbar ist, wobei das optische Teilerelement (5) bezüglich Reflexion und Transmission für zueinander komplementäre Licht-Polarisationen ausgelegt ist,
**dadurch gekennzeichnet, daß** das optische Teilerelement (5) bezüglich Reflexion und Transmission zusätzlich für zueinander komplementäre Wellenlängenbereiche ausgelegt ist, wobei der in das Bildübertragungssystem (2) eingekoppelte Wellenlängenbereich im kurzwelligen und der in das in das Beobachtungssystem (4) ausgekoppelte Wellenlängenbereich im daran anschließenden längerwelligen Spektralbereich liegen.

3. Endoskop nach Anspruch 2, **dadurch gekennzeichnet, daß** distalseitig dem Objektiv (3) ein λ/4-Plättchen (7) vorgeschaltet ist.

4. Endoskop nach Anspruch 1 oder 2, **dadurch gekennzeichnet, daß** dem Teilerelement (5) auf der Beleuchtungsseite zusätzlich ein Filterelement (8) als Polarisator und auf der Beobachtungsseite zusätzlich ein Filterelement (9) als Analysator zugeordnet sind.

5. Endoskop nach Anspruch 1 oder 2, **dadurch gekennzeichnet, daß** dem Teilerelement (5) auf der Beleuchtungsseite zusätzlich ein Filterelement (8) als Spektralfilter für den kurzwelligen Spektralbereich und auf der Beobachtungsseite zusätzlich ein Filterelement (9) als Spektralfilter für den längerwelligen Spektralbereich zugeordnet sind.

6. Endoskop nach Anspruch 1 oder 2, **dadurch gekennzeichnet, daß** das Bildübertragungssystem (2) als Multifaser-Bildleitersystem mit planen Endflächen (18, 23) ausgebildet ist.

7. Endoskop nach Anspruch 6, **dadurch gekennzeichnet, daß** das Multifaser-Bildleitersystem aus Monomode-Fasern besteht.

8. Endoskop nach Anspruch 1 oder 2, **dadurch gekennzeichnet, daß** das Bildübertragungssystem (2) aus Stablinsen (28) mit gekrümmten Endflächen besteht.

9. Endoskop nach Anspruch 1 oder 2, **dadurch gekennzeichnet, daß** das Bildübertragungssystem (2) aus Gradientenindex-Stablinsen mit planen Endflächen besteht.

10. Endoskop nach einem der vorhergehenden Ansprüche, **dadurch gekennzeichnet, daß** ein zusätzliches faseroptisches Beleuchtungssystem (10) zur Aufhellung des Hohlraumes im Weißlicht (11) vorgesehen ist und proximalseitig zwischen das Bildübertragungssystem (2) und das Teilerelement (5) ein Neutralteiler (13) zur Auskopplung eines Teiles des zurückkommenden Lichts für eine zusätzliche Beobachtung (12) eingefügt ist.

11. Endoskop nach Anspruch 1 oder 2, **dadurch gekennzeichnet, daß** das optische Teilerelement (5) aus zwei gleichseitigen Rechtwinkelprismen (19) zu einem Würfel zusammengesetzt ist, bei dem die Hypotenusenfläche eines der Rechtwinkelprismen (19) mit einer spektralen und polarisationsoptischen oder einer polarisationsoptischen Teilerschicht (20) belegt ist und wobei je eine geeignete Kathetenfläche der Beleuchtungseinheit (1), dem Bildübertragungssystem (2) und dem Beobachtungssystem (4) zugeordnet ist.

12. Endoskop nach Anspruch 11, **dadurch gekennzeichnet, daß** den Kathetenflächen zwischen Beleuchtungseinheit (1) und Beobachtungssystem (4) ergänzende polarisationsoptische oder spektrale Filterelemente (8, 9) zugeordnet sind.

13. Endoskop nach Anspruch 11, **dadurch gekennzeichnet, daß** als Beobachtungssystem (4) ein Flächen-Bildsensor (25) mit integriertem halbleiterbasierten Ladungs- bzw. Elektronenvervielfacher vorgesehen und einer Kathetenfläche des Teilerelements (5) zugeordnet ist.

14. Endoskop nach Anspruch 13, **dadurch gekennzeichnet, daß** dem Flächen-Bildsensor (25) ein Kühlelement zugeordnet ist.

15. Endoskop nach Anspruch 11, **dadurch gekennzeichnet, daß** als Beleuchtungseinheit (1) eine Laserdiodenmatrix vorgesehen und einer Kathetenfläche des Teilerelements (5) zugeordnet ist.

16. Endoskop nach Anspruch 1 oder 2, **dadurch gekennzeichnet, daß** die Beleuchtungseinheit (1), das Teilerelement (5) und das Beobachtungssystem (4) in einer Gehäuseeinheit (14) zusammengefaßt sind, die mit einem Adapter (22) zum auswechselbaren Ansetzen des Bildübertragungssystems (2) an die zugehörige Kathetenfläche des Teilerelements (5) versehen ist.

17. Endoskop nach Anspruch 16, **dadurch gekennzeichnet, daß** das Teilerelement (5) in der Gehäuseeinheit (14) auswechselbar angeordnet ist.

18. Endoskop nach Anspruch 2, **dadurch gekennzeichnet, daß** das Teilerelement (5) beleuchtungsseitig für den Wellenlängenbereich im nahen UV und beobachtungsseitig für den sichtbaren Wellenlängenbereich vorgesehen ist.

19. Endoskop nach Anspruch 18, **dadurch gekennzeichnet, daß** der beleuchtungsseitige Wellenlängenbereich bis ca. 430 nm reicht und der beobachtungsseitige Wellenlängenbereich bei ca. 430 nm beginnt.

20. Endoskop nach Anspruch 18, **dadurch gekennzeichnet, daß** der beleuchtungsseitige Wellenlängenbereich bis ca. 450 nm reicht und der beobachtungsseitige Wellenlängenbereich bei ca. 450 nm beginnt.

21. Endoskop nach Anspruch 1 oder 2, **dadurch gekennzeichnet, daß** die Beleuchtungseinheit (1) eine Xenon-, Quecksilberdampf- oder Halid-Lampe zur Erzeugung einer inkohärenten Beleuchtung enthält.

22. Endoskop nach Anspruch 1 oder 2, **dadurch gekennzeichnet, daß** die Beleuchtungseinheit (1) ein Lasersystem als Strahlungsquelle enthält.

## Claims

1. Endoscope for illuminating and observing object fields (6) in cavities, with an illumination unit (1) and an endoscopic image transmission system (2) to which are assigned on the distal end an objective (3) and on the proximal end an eyepiece optics or camera optics as observation system (4), which on the proximal side contains an optical splitter element (5) which is inserted between the illumination unit (1), the image transmission system (2) and the observation system (4) such that the illuminating light produced by the illumination unit (1) can be coupled into the image transmission system (2), the optical splitter element (5) being designed, with respect to reflection and transmission, for mutually complementary light polarizations, **characterized in that** on the distal side a λ/4 plate (7) is connected upstream of the objective (3).

2. Endoscope for illuminating and observing object fields (6) in cavities, with an illumination unit (1) and an endoscopic image transmission system (2) to which are assigned on the distal side an objective (3) and on the proximal side an eyepiece optics or camera optics as observation system (4), which on the proximal side contains an optical splitter element (5) which is inserted between the illumination unit (1), the image transmission system (2) and the observation system (4) such that the illuminating light produced by the illumination unit (1) can be coupled into the image transmission system (2), the optical splitter element (5) being designed, with respect to reflection and transmission, for mutually complementary light polarizations, **characterized in that** the optical splitter element (5) is additionally designed, with respect to reflection and transmission, for mutually complementary wavelength ranges, with the wavelength range which is coupled into the image transmission system (2) being in the shortwave spectral range, and the wavelength range coupled out into the observation system (4) being in the longer-wave spectral range which follows the shortwave spectral range.

3. Endoscope according to Claim 2, **characterized in that** on the distal side, a λ/4 plate (7) is connected upstream of the objective (3).

4. Endoscope according to Claim 1 or 2, **characterized in that**, on the illumination side, additionally a filter element (8) as polarizer and, on the observation side, additionally a filter element (9) as analyser are assigned to the splitter element (5).

5. Endoscope according to Claim 1 or 2, **characterized in that**, on the illumination side, additionally a filter element (8) as spectral filter for the shortwave spectral range and, on the observation side, additionally a filter element (9) as spectral filter for the longer-wave spectral range are assigned to the splitter element (5).

6. Endoscope according to Claim 1 or 2, **characterized in that** the image transmission system (2) is designed as a multifibre image conductor system with planar end faces (18, 23).

7. Endoscope according to Claim 6, **characterized in that** the multifibre image conductor system comprises monomode fibres.

8. Endoscope according to Claim 1 or 2, **characterized in that** the image transmission system (2) comprises rod lenses (28) with curved end faces.

9. Endoscope according to Claim 1 or 2, **characterized in that** the image transmission system (2) comprises gradient index rod lenses with planar end faces.

10. Endoscope according to one of the preceding claims, **characterized in that** an additional fibre-optic illuminating system (10) for lighting the cavity using white light (11) is provided, and a neutral splitter (13) for coupling out a portion of the returning light is inserted on the proximal side between the image transmission system (2) and the splitter element (5) for additional observation (12).

11. Endoscope according to Claim 1 or 2, **characterized in that** the optical splitter element (5) is composed of two equilateral right-angle prisms (19) to form a cube in which the face of the hypotenuse of one of the right-angle prisms (19) is covered by a spectral and polarization-optical or a polarization-optical splitter layer (20) and wherein in each case one suitable cathetus face is assigned to the illumination unit (1), the image transmission system (2) and the observation system (4).

12. Endoscope according to Claim 11, **characterized in that** supplementary polarization-optical or spectral filter elements (8, 9) are assigned to the cathetus faces between illumination unit (1) and observation system (4).

13. Endoscope according to Claim 11, **characterized in that**, as observation system (4), a surface image sensor (25) with integrated semiconductor-based charge and electron multiplier is provided and assigned to a cathetus face of the splitter element (5).

14. Endoscope according to Claim 13, **characterized in that** a cooling element is assigned to the surface image sensor (25).

15. Endoscope according to Claim 11, **characterized in that**, as illumination unit (1), a laser diode matrix is provided and assigned to a cathetus face of the splitter element (5).

16. Endoscope according to Claim 1 or 2, **characterized in that** the illumination unit (1), the splitter element (5) and the observation system (4) are combined in a housing unit (14) which is provided with an adapter (22) for replaceably fitting the image transmission system (2) on the associated cathetus face of the splitter element (5).

17. Endoscope according to Claim 16, **characterized in that** the splitter element (5) is replaceably arranged in the housing unit (14).

18. Endoscope according to Claim 2, **characterized in that** the splitter element (5) is provided on the illumination side for the near-UV wavelength range, and on the observation side for the visible wavelength range.

19. Endoscope according to Claim 18, **characterized in that** the illumination-side wavelength range extends to about 430 nm, and the observation-side wavelength range starts at about 430 nm.

20. Endoscope according to Claim 18, **characterized in that** the illumination-side wavelength range extends to about 450 nm and the observation wavelength range starts at about 450 nm.

21. Endoscope according to Claim 1 or 2, **characterized in that** the illumination unit (1) contains a xenon lamp, mercury vapour lamp or halide lamp for producing incoherent illumination.

22. Endoscope according to Claim 1 or 2, **characterized in that** the illumination unit (1) contains a laser system as radiation source.

## Revendications

1. Endoscope pour éclairer et pour observer des zones d'objets (6) dans des cavités avec une unité d'éclairage (1) et un système endoscopique de transfert d'images (2) auquel sont associés un objectif (3) côté distal et une optique d'oculaire ou de caméra, côté proximal, en tant que système d'observation (4) comprenant côté proximal un élément de séparation optique (5), qui est inséré entre l'unité d'éclairage (1), le système de transfert d'images (2) et le système d'observation (4), de sorte que la lumière d'éclairage générée par l'unité d'éclairage (1) soit injectable dans le système de transfert d'images (2), l'élément de séparation optique (5) étant conçu au niveau de la réflexion et de la transmission pour des polarisations de lumières mutuellement complémentaires, **caractérisé en ce qu'**une lamelle λ/4 (7) est montée en amont de l'objectif (3), côté distal.

2. Endoscope pour éclairer et pour observer des zones d'objets (6) dans des cavités avec une unité d'éclairage (1) et un système endoscopique de transfert d'images (2) auquel sont associés un objectif (3) côté distal et une optique d'oculaire ou de caméra, côté proximal, en tant que système d'observation (4) comprenant côté proximal un élément de séparation optique (5), qui est inséré entre l'unité d'éclairage (1), le système de transfert d'images (2) et le système d'observation (4), de sorte que la lumière d'éclairage générée par l'unité d'éclairage (1) soit injectable dans le système de transfert d'images (2), l'élément de séparation optique (5) étant conçu au niveau de la réflexion et de la transmission pour des polarisations de lumières mutuellement complémentaires, **caractérisé en ce que** l'élément de séparation optique (5) est en outre conçu au niveau de la réflexion et de la transmission pour des gammes de longueurs d'ondes mutuellement complémentaires, la gamme de longueurs d'ondes injectée dans le système de transfert d'images (2) se situant dans le domaine spectral à faibles longueurs d'ondes et la gamme de longueurs d'ondes extraite dans le système d'observation (4) se situant dans le domaine spectral à ondes plus longues qui y fait suite.

3. Endoscope selon la revendication 2, **caractérisé en ce qu'**une lamelle λ/4 (7) est montée en amont de l'objectif (3), côté distal.

4. Endoscope selon la revendication 1 ou 2, **caractérisé en ce que** côté éclairage, un élément de filtrage (8) en tant que polarisateur et côté observation, un élément de filtrage (9) en tant qu'analyseur sont associés en supplément à l'élément de séparation (5).

5. Endoscope selon la revendication 1 ou 2, **caractérisé en ce que** côté éclairage, un élément de filtrage (8) en tant que filtre spectral pour le domaine spectral à faibles longueurs d'ondes et côté observation, un élément de filtrage (9) en tant que filtre spectral pour le domaine spectral à ondes plus longues sont associés en supplément à l'élément de séparation (5).

6. Endoscope selon la revendication 1 ou 2, **caractérisé en ce que** le système de transfert d'images (2) est conçu sous la forme d'un système de conduction d'images multifibres avec des surfaces d'extrémité planes (18, 23).

7. Endoscope selon la revendication 6, **caractérisé en ce que** le système de conduction d'images multifibres consiste en des fibres monomode.

8. Endoscope selon la revendication 1 ou 2, **caractérisé en ce que** le système de transfert d'images (2) consiste en des lentilles barreaux (28) avec des surfaces d'extrémité recourbées.

9. Endoscope selon la revendication 1 ou 2, **caractérisé en ce que** le système de transfert d'images (2) consiste en des lentilles barreaux à indice de gradient, avec des surfaces d'extrémité planes.

10. Endoscope selon l'une quelconque des revendications précédentes, **caractérisé en ce qu'**un système d'éclairage à fibres optiques (10) supplémentaire est prévu pour éclairer la cavité d'une lumière blanche (11) et **en ce que** côté proximal, entre le système de transfert d'images (2) et l'élément de séparation (5) est inséré un séparateur neutre (13), pour extraire une partie de la lumière en retour, pour une observation supplémentaire (12).

11. Endoscope selon la revendication 1 ou 2, **caractérisé en ce que** l'élément de séparation optique (5) est assemblé en un dé composé de deux prismes équilatéraux à angles droits (19), sur lequel la surface de l'hypoténuse de l'un des prismes à angles droits (19) est occupée par une couche de séparation (20) spectrale et optique de polarisation ou optique de polarisation et chaque fois une surface appropriée du côté de l'angle droit de l'unité d'éclairage (1) étant associée au système de transfert d'images (2) et au système d'observation (4).

12. Endoscope selon la revendication 11, **caractérisé en ce que**, entre l'unité d'éclairage (1) et le système d'observation (4), des éléments de filtrage (8, 9) optiques de polarisation ou spectraux complémentaires sont associés aux surfaces du côté de l'angle droit.

13. Endoscope selon la revendication 11, **caractérisé en ce qu'**un capteur d'images superficiel (25) avec multiplicateur de charge ou d'électrons intégré, basé semi-conducteurs est prévu en tant que système d'observation (4) et associé à une surface du côté de l'angle droit de l'élément de séparation optique (5).

14. Endoscope selon la revendication 13, **caractérisé en ce qu'**un élément de refroidissement est associé au capteur d'image superficiel (25).

15. Endoscope selon la revendication 11, **caractérisé en ce qu'**une matrice à diode laser est prévue en tant qu'unité d'éclairage (1) et associée à une surface du côté de l'angle droit de l'élément de séparation (5).

16. Endoscope selon la revendication 1 ou 2, **caractérisé en ce que** l'unité d'éclairage (1), l'élément de séparation (5) et le système d'observation (4) sont regroupés dans une unité de boîtier (14) qui est munie d'un adaptateur (22) pour le montage interchangeable du système de transfert d'images (2) sur la surface du côté de l'angle droit associée de l'élément de séparation.

17. Endoscope selon la revendication 16, **caractérisé en ce que** l'élément de séparation (5) est disposé de façon interchangeable dans l'unité de boîtier (14).

18. Endoscope selon la revendication 2, **caractérisé en ce que** l'élément de séparation (5) est prévu côté éclairage pour la gamme des longueurs d'ondes proches des UV et côté observation pour la gamme des longueurs d'ondes visibles.

19. Endoscope selon la revendication 18, **caractérisé en ce que** la gamme de longueurs d'ondes côté éclairage va jusqu'à environ 430 nm et que la gamme de longueurs d'ondes côté observation commence à environ 430 nm.

20. Endoscope selon la revendication 18, **caractérisé en ce que** la gamme de longueurs d'ondes côté éclairage va jusqu'à environ 450 nm et que la gamme de longueurs d'ondes côté observation commence à environ 450 nm.

21. Endoscope selon la revendication 1 ou 2, **caractérisé en ce que** l'unité d'éclairage (1) contient une lampe au xénon, à vapeur de mercure, ou à haloïde pour créer un éclairage incohérent.

22. Endoscope selon la revendication 1 ou 2, **caractérisé en ce que** l'unité d'éclairage (1) contient un système de laser en tant que source de rayonnement.
